# EUROPEAN PATENT SPECIFICATION

(11) **EP 4 106 553 B1**
(45) Date of publication and mention of the grant of the patent: **15.07.2026**
(21) Application number: 21707630.6
(22) Date of filing: 17.02.2021
(51) Int. Cl.: A23L 33/105, A23L 33/15, A23L 33/155, A61K 9/14, A61K 31/047, A61K 31/07, A61K 31/122, A61K 31/59

(54) **STABLE FAT-SOLUBLE VITAMIN POWDERS**
STABILE FETTLÖSLICHE VITAMINE PULVER
POUDRES STABLES DE VITAMINES LIPOSOLUBLES

(30) Priority: 18.02.2020 EP 20157973
(43) Date of publication of application: 28.12.2022
(73) Proprietor: BASF SE, 67056 Ludwigshafen am Rhein (DE)
(72) Inventor: MUSAEUS, Nina, 2750 Ballerup (DK); FELDTHUSEN JENSEN, Jesper, 68623 Lampertheim (DE); SOENDERGAARD, Claus, 2750 Ballerup (DK); SUBINYA ALBRICH, Mireia, 67056 Ludwigshafen (DE); CHU, Fangfang, 68623 Lampertheim (DE)
(74) Representative: BASF IP Association
(86) International application number: PCT/EP2021/053827
(87) International publication number: WO 2021/165288

(56) References cited:
- EP-A1- 1 938 807
- CN-A- 103 735 532
- CN-A- 106 579 407
- US-A1- 2011 052 680

## Description

The synthetic antioxidant BHT (butyl hydroxy toluene) has a negative reputation both from a health and an environmental perspective. Therefore, substitution of BHT with alternative antioxidants is targeted to provide sustainable vitamin A powder products with state-of-the-art performance. Staple foods such as oil, flour, sugar, rice and condiments are fortified with vitamin A. Vitamin A powders are used for fortifying e.g. flour and sugar. The test conditions in the accelerated tests used for approving a vitamin A powder for flour or sugar application are very harsh. Until now only vitamin A powders containing the very effective antioxidant BHT were able to pass the tests. Currently all vitamin A powders on the market intended for flour or sugar fortification contain BHT. Formulations comprising carotenoids and an antioxidant system which is BHT-free are disclosed in e.g., CN 106579407 A and CN 103735532 A.

The present invention relates to stable fat-soluble vitamin or carotenoid powders not containing BHT but still having similar stability in flour and sugar premixes stored under accelerated conditions as current BHT-containing products for these applications.

It was surprisingly found, that a combination of sodium-ascorbate and tocopherol (DL-α-tocopherol or natural mixed tocopherol) as antioxidants in a powder formulation comprising a fat-soluble vitamin or a carotenoid, a hydrocolloid and a starch hydrolysate provided similar stability in flour and sugar premix stored under accelerated conditions as current commercial products containing BHT.

It was not to be foreseen by the person skilled in the art that a combination of sodium-ascorbate and tocopherol according to the present invention and the use of it would solve above mentioned issues.

The present invention, which is defined by the claims, relates to:
- A powderous BHT-free formulation comprising 1 to 25wt.-% of total weight of the powder of a fat soluble vitamin or a carotenoid, 9.25 to 25wt.-% of total weight of the powder of a hydrocolloid, 20 to 89wt.-% of total weight of the powder of a starch hydrolysate and 0.75 to 12wt.-% of total weight of the powder of tocopherol and sodium ascorbate, optionally 0 to 20wt.- % of total weight of the powder of carrier oil, optionally 0 to 50wt.-% of total weight of the powder of a mixture of powdering and anti-caking agent and optionally 0 to 5wt.-% of total weight of the powder of residual water, whereby the weight ratio of sodium ascorbate to tocopherol is between 1 and 6, preferred between 1.5 and 6.

It also relates to:
- The use of the above formulation for staple food fortification.

Examples of carotenoids are β-carotene, lutein, lutein ester or lycopene and mixtures thereof.

Examples of fat-soluble vitamins are vitamin A, vitamin D, Vitamin K and/or its derivatives or mixtures thereof. Preferred is vitamin A and/or its derivative, more preferred vitamin A esters and here especially the vitamin A palmitate.

According to the invention the content of vitamin-A esters is from 5 to 25wt.-% of the formulation, preferably from 10 to 22 wt.-%, more preferably from 12 to 20 wt.-% of the total weight of the powderous formulation. The preferred vitamin A ester is vitamin A palmitate.

As a further component of the powderous formulation of the invention it comprises a combination of two antioxidants sodium ascorbate and tocopherol, whereby the term tocopherol encompasses DL-α-tocopherol and mixed tocopherol and whereby the weight-ratio of sodium ascorbate to tocopherol is between 1 and 6, preferably between 1.5 and 6. The content of sodium-ascorbate and tocopherol on the formulation is from 0.75 to 12wt.-% of the formulation, preferably from 2 to 8 wt.-%, more preferably from 2 to 4 wt.-%.

The powderous formulation contains further a hydrocolloid as emulsifier and matrix component.

According to the invention the content of said at least one hydrocolloid is from 9.25 to 25wt.-% of the formulation, preferably from 10 to 22wt.-%, more preferably from 12-20wt.-% of the total weight of the powderous formulation.

Examples of hydrocolloids are polysaccharides, gelatin of low bloom, medium bloom or high bloom from fish, pork or bovine, caseines/caseinates and other proteinaceous hydrocolloids.

Preferred according to the invention are polysaccharides.

The term polysaccharide as used herein includes xanthan gum, gum acacia, pectin, guar gum, caroub gum, alginates, celluloses, cellulose derivatives, such as starch and starch derivatives. Preferred polysaccharides according to the present invention are gum acacia, starch, starch derivatives; especially preferred are gelatinized starch and modified food starch and here particularly preferred are modified food starches.

The term "modified food starch" as used herein relates to modified starches that are made from starches substituted by known chemical methods with hydrophobic moieties. For example starch may be treated with cyclic dicarboxylic acid anhydrides such as succinic and/or glutaric anhydrides, substituted with an alkyl or alkenyl hydrocarbon group.

A preferred modified starch is starch sodium octenyl succinate (OSA-starch). OSA-starch as used herein denotes any starch (from any natural source such as corn, wheat, tapioca, potato or synthesized) that was treated with octenyl succinic anhydride. The degree of substitution, i.e. the number of esterified hydroxyl groups with regard to the total number of hydroxyl groups usually varies in a range of from 0.1% to 10%, preferably in a range of from 0.5% to 5%, more preferably in a range of from 2% to 4%.

OSA-starch is commercially available e.g. from Ingredion under the trade names HiCap 100, Capsul HF, Capsul HS, Purity Gum 2000, UNI-PURE, HYLON VII from Roquette Frères; from Cargill under the trade name C*EmCap or from Tate & Lyle. HiCap 100, Capsul HF and Capsul HS are especially preferred, mostly preferred are Capsul HF and Capsul HS.

A further component of the formulation according to the present invention are the "starch hydrolysates", which as used herein denote dextrins, maltodextrins and glucose syrup and have the function as matrix component and plasticizer. Preferred are starch hydrolysates with a DE-value of 10 to 30, more preferred are hydrolysates with a DE-value of 15 to 25, most preferred are hydrolysates with a DE-value of 17 to 20.

The DE-value characterizes the reducing capacity based on the reducing capacity of anhydrous dextrose and is determined by the DIN 10 308 method, edition 5.71, of the Deutsche Normenausschuss Lebensmittel und landwirtschaftliche Produkte.

According to the invention the content of said at least one starch hydrolysate is from 20 to 89,75wt.-% of the formulation, preferably from 25 to 78wt.-%, more preferably from 30 to 74wt.- % of the total weight of the powderous formulation.

The powderous formulation according to the invention may further contain, depending on the drying process additional constituents such as i) native starches as a powdering agent and ii) anti-caking agents, e.g. tri-calcium phosphate and silicates, such as silicon dioxide or sodium aluminium silicate, which embed the powders according to the invention and which content in total is from 0 to 50% of the total weight of the powder. Furthermore carrier oil such as sunflower oil, corn oil, MCT oil (medium chain triglycerides) or other vegetable oils are optionally present in the formulation in an amount of 0 to 20% of the total weight of the powder. Residual water (residual humidity) which content in total is 0 to 5% of the total weight of the powder can also be present in the powder after the drying process.

The invention is further illustrated by the following examples.

### Examples:

Measuring stability of the vitamin-A palmitate formulation in flour (Flour test, based on USAID - Moist Flour Test CSB13)
Mix 0.2 g vitamin A palmitate powder produced ac to example 1-14 with 5 g flour having a moisture content of 13.5 to 14.5%. Store the mixture in a closed vial at 45°C for 21 days. Analyse the vitamin A content in the mixture at time point zero (initial value) and after 10 and 21 days of storage. Use one vial for each single determination. Calculate the remaining vitamin A in percentages of the initial value at each time point.
Measuring stability of the vitamin-A palmitate formulation in sugar (sugar premix ad modum IN-CAP (Instituto Nutricion Centro America y Panama).)

Prepare the following blends:
1) Sugar blend: 22g vitamin A palmitate powder produced according to example 1 to 14 and 75g sucrose.
2) Antioxidant/oil blend: 160mg Grindox^{®} 1020 and 29,76g coconut oil heated to 37°C.

### Sugar premix:

Take out 1,49 g of the antioxidant/oil blend and mix it into the sugar blend. Mix until the oil is evenly distributed throughout the sugar. Store the sugar premix in PE-bags at 40°C/75 % RH for 3 months. Analyse the vitamin A content at time point zero (initial value) and after 1, 2 and 3 months storage. Calculate the remaining vitamin A in percentages of the initial value at each time point.

### Example 1

600 g Capsul HS, 1479 g Glucidex 19 and 78 g sodium ascorbate were dissolved during stirring in 1430 g of water at 60-65°C. A mixture of 600 g vitamin A palmitate oil (1,7 Mio IU/g) and 27 g D,L-α-tocopherol was added to the aqueous solution during stirring and emulsified until the average oil droplet size d(0.5) was less than 1 µm (measured by Malvern Mastersizer 3000). The viscosity was adjusted with water and the emulsion was sprayed into native corn starch (powdering agent) containing tricalcium phosphate (TCP) (anti-caking agent). The formed particles were dried in air at 25-150°C until the water content in the powder was below 5%.

The powder produced according to example 1 had the following composition [weight-%]:

| | |
|---|---|
| Maltodextrin | 46.4 |
| Modified food starch | 18.8 |
| Vitamin A palmitate (1,7 Mio IU/g) | 18.8 |
| Sodium ascorbate | 2.4 |
| DL-α-tocopherol | 0.8 |
| Corn starch + TCP | 8.0 |
| Residual water (residual humidity) | 4.8 |

The examples 2 to 5 (Table 1) were produced according to example 1, with the exception that the DL-α-tocopherol amount was kept constant, while the sodium ascorbate amount was reduced.

**Table 1:**

| Example | sodium ascorbate [g] | DL-α-tocopherol | Weight ratio sodium-ascorbate to DL-α-tocopherol |
|---|---|---|---|
| 1 | 78 | 27 | 2.,89 |
| 2 | 60 | 27 | 2.22 |
| 3 | 30 | 27 | 1.11 |
| 4 | 15 | 27 | 0.56 |
| 5 | 0 | 27 | 0.0 |

The products produced according to examples 1-5 were tested in the flour test (Table 2) and in the sugar premix test (Table 3). Commercial vitamin A palmitate products stabilized with BHT were used as references in the tests

**Table 2**

| Example | sodium-ascorbate [g] | DL-α-tocopherol [g] | Weight ratio sodium-ascorbate to DL-α-tocopherol | Initial [% remaining of initial] | 10 days [% remaining of initial] | 21 days [% remaining of initial] |
|---|---|---|---|---|---|---|
| **1** | 78 | 27 | 2.89 | 100 | 93 | 87 |
| **2** | 60 | 27 | 2.22 | 100 | 88 | 86 |
| **3** | 30 | 27 | 1.11 | 100 | 93 | 77 |
| **4** | 15 | 27 | 0.56 | 100 | 89 | 37 |
| **5** | 0 | 27 | - | 100 | 34 | 18 |
| Reference: Commercial vitamin A palmitate 250 containing BHT from BASF | | | | 100 | 91 | 82 |

**Table 3**

| Example | sodium-ascorbate [g] | DL-α-tocopherol [g] | Weight ratio sodium-ascorbate to DL-α-tocopherol | Initial [% remaining of initial] | 1 month [% remaining of initial] | 2 months [% remaining of initial] | 3 months [% remaining of initial] |
|---|---|---|---|---|---|---|---|
| **1** | 78 | 27 | 2.89 | 100 | 90 | 83 | 73 |
| **2** | 60 | 27 | 2.22 | 100 | 88 | 83 | 60 |
| **3** | 30 | 27 | 1.11 | 100 | 89 | 66 | 38 |
| **4** | 15 | 27 | 0.56 | 100 | 76 | 47 | N/A |
| **5** | 0 | 27 | - | 100 | 64 | 43 | N/A |
| Reference: Commercial vitamin A palmitate 250 containing BHT from BASF | | | | 100 | 80 | 73 | 61 |

**Table 2 and 3** show that a combination of DL-α-tocopherol and sodium-ascorbate increases the stability of the vitamin-A palmitate-formulation in the flour and in the sugar premix. Furthermore, with this antioxidant combination it is possible to make a formulation with similar stability in flour and sugar premix as the current commercially available BHT-containing formulation.

### Example 6

600 g Capsul HS, 1479 g Glucidex 19 and 60 g sodium ascorbate were dissolved in 1430 g of water at 60-65°C during stirring. A mixture of 600 g vitamin A palmitate oil (1,7 Mio IU/g) and 39 g Covi-Ox-T-70 mixed tocopherols, 70% concentrate) was added to the aqueous solution during stirring and emulsified until the average oil droplet size d(0.5) was less than 1 µm (measured by Malvern Mastersizer 3000). The viscosity was adjusted with water and the emulsion was sprayed into native corn starch (powdering agent) containing tricalcium phosphate (TCP) (anti-caking agent). The formed particles were dried in air at 25-150°C until the water content in the powder was below 5%.

The powder produced according to example 6 had the following composition [weight-%]:

| | |
|---|---|
| Maltodextrin | 49.6 |
| Modified food starch | 20.0 |
| Vitamin A palmitate (1,7 Mio IU/g) | 20.0 |
| Sodium ascorbate | 2.0 |
| Mixed-tocopherol, 70% concentrate | 1.3 |
| Corn starch + TCP | 2.2 |
| Residual water (residual humidity) | 4.9 |

The examples 7 to 10 (Table 4) were produced according to example 6, with the exception that the mixed-tocopherol amount was kept constant, while the sodium ascorbate amount was reduced.

**Table 4:**

| Example | sodium ascorbate [g] | mixed-tocopherol (70% concentrate) [g] | Weight ratio sodium-ascorbate to mixed-tocopherol (100%) |
|---|---|---|---|
| 6 | 60 | 39 | 2.2 |
| 7 | 45 | 39 | 1.65 |
| 8 | 30 | 39 | 1.10 |
| 9 | 15 | 39 | 0.55 |
| 10 | 0 | 39 | 0.0 |

The products produced according to examples 6-10 were tested in the flour test (Table 5) and in the sugar premix test (Table 6). Commercial vitamin A palmitate products stabilized with BHT were used as references in the tests.

**Table 5**

| Example | Sodium ascorbate [g] | mixed tocopherol (70%) [g] | Weight ratio sodium-ascorbate to mixed tocopherol ("100%") | Initial [% remaining of initial] | 10 days [% remaining of initial] | 21 days [% remaining of initial] |
|---|---|---|---|---|---|---|
| 6 | 60 | 39 | 2.2 | 100 | 89 | 84 |
| 7 | 45 | 39 | 1.65 | 100 | 87 | 86 |
| 8 | 30 | 39 | 1.10 | 100 | 92 | 80 |
| 9 | 15 | 39 | 0.55 | 100 | 90 | 72 |
| 10 | 0 | 39 | - | 100 | 57 | 37 |
| Reference: Commercial VAP 250 containing BHT from BASF | | | | 100 | 91 | 82 |

**Table 6**

| Example | Sodium ascorbate [g] | Mixed tocopherol (70%) [g] | Weight ratio sodium-ascorbate to mixed tocopherol ("100") | Initial [% remaining of initial] | 1 month [% remaining of initial] | 2 months [% remaining of initial] | 3 months [% remaining of initial] |
|---|---|---|---|---|---|---|---|
| 6 | 60 | 39 | 2.2 | 100 | 86 | 81 | 66 |
| 7 | 45 | 39 | 1.65 | 100 | 87 | 83 | 55 |
| 8 | 30 | 39 | 1.10 | 100 | 87 | 57 | 42 |
| 9 | 15 | 39 | 0.55 | 100 | 82 | 52 | N/A |
| 10 | 0 | 39 | - | 100 | 49 | 39 | N/A |
| Reference: Commercial vitamin A palmitate 250 containing BHT from BASF | | | | 100 | 80 | 73 | 61 |

**Table 5 and 6** show that a combination of mixed tocopherols and sodium ascorbate increases the stability of the vitamin A palmitate formulation in the flour and in the sugar premix. Furthermore, with this antioxidant combination it is possible to make a formulation with similar stability in flour and sugar premix as the current commercially available BHT-containing formulation.

### Example 11

600 g Capsul HS, 1766 g Glucidex 19 and 78 g sodium ascorbate were dissolved in 1430 g of water at 60-65°C during stirring. A mixture of 600 g vitamin A palmitate oil (1,7 Mio IU/g) and 40.5 g DL-α-tocopherol was added to the aqueous solution during stirring and emulsified until the average oil droplet size d(0.5) was less than 1 µm (measured by Malvern Mastersizer 3000). The viscosity was adjusted with water and the emulsion was sprayed into native corn starch (powdering agent) containing tricalcium phosphate (TCP) (anti-caking agent). The formed particles were dried in air at 25-150°C until the water content in the powder was below 5%.

The powder produced according to example 11 had the following composition [weight-%]:

| | |
|---|---|
| Maltodextrin | 52.3 |
| Modified food starch | 17.8 |
| Vitamin A palmitate (1,7 Mio IU/g) | 17.8 |
| Sodium ascorbate | 2.3 |
| DL-α-tocopherol | 1.2 |
| Corn starch + TCP | 4.0 |
| Residual water | 4.6 |

The examples 12 to 14 (Table 7) were produced according to example 11, with the exception that the sodium ascorbate amount was kept constant, while the DL-α-tocopherol amount was reduced.

**Table 7:**

| Example | sodium ascorbate [g] | DL-α-tocopherol | Weight ratio sodium-ascorbate to DL-α-tocopherol |
|---|---|---|---|
| 11 | 78 | 40.5 | 1.93 |
| 12 | 78 | 27 | 2.89 |
| 13 | 78 | 13.5 | 5.78 |
| 14 | 78 | 0 | N/A |

The products produced according to example 5 and 11 to 14 were tested in the flour test (Table 8) and in the sugar premix test (Table 9). Commercial vitamin A palmitate products stabilized with BHT were used as references in the tests.

**Table 8**

| Example | sodium-ascorbate [g] | DL-α tocopherol [g] | Weight ratio sodium-ascorbate to DL-α tocopherol | Initial [% remaining of initial] | 10 days [% remaining of initial] | 21 days [% remaining of initial] |
|---|---|---|---|---|---|---|
| 11 | 78 | 40,5 | 1,93 | 100 | 88 | 81 |
| 12 | 78 | 27 | 2,89 | 100 | 90 | 85 |
| 13 | 78 | 13,5 | 5,78 | 100 | 92 | 87 |
| 14 | 78 | 0 | N/A | 100 | 50 | 24 |
| 5 | 0 | 27 | N/A | 100 | 34 | 18 |
| Reference: Commercial vitamin A palmitate 250 containing BHT from BASF | | | | 100 | 91 | 82 |

**Table 9**

| Example | sodium-ascorbate [g] | DL-α-tocopherol [g] | Weight ratio sodium-ascorbate to DL-α-tocopherol | Initial [% remaining of initial] | 1 month [% remaining of initial] | 2 months [% remaining of initial] | 3 months [% remaining of initial] |
|---|---|---|---|---|---|---|---|
| 11 | 78 | 40.5 | 1.93 | 100 | 87 | 79 | 53 |
| 12 | 78 | 27 | 2.89 | 100 | 87 | 85 | 66 |
| 13 | 78 | 13.5 | 5.78 | 100 | 90 | 84 | 66 |
| 14 | 78 | 0 | N/A | 100 | 63 | 40 | 26 |
| 5 | 0 | 27 | N/A | 100 | 64 | 43 | N/A |
| Reference: Commercial vitamin A palmitate 250 containing BHT from BASF | | | | 100 | 80 | 73 | 61 |

## Claims

1. Powderous BHT-free formulation comprising 1 to 25wt.-% of total weight of the powder of a fat soluble vitamin or a carotenoid, 9.25 to 25wt.-% of total weight of the powder of a hydrocolloid, 20 to 89wt.-% of total weight of the powder of a starch hydrolysate and 0.75 to 12wt.-% of total weight of the powder of tocopherol and sodium ascorbate, optionally 0 to 20wt.-% of total weight of the powder of carrier oil, optionally 0 to 50wt.-% of total weight of the powder of a mixture of powdering and anti-caking agent and optionally 0 to 5wt.-% of total weight of the powder of residual water, whereby the weight ratio of sodium ascorbate to tocopherol is between 1 and 6, preferred between 1.5 and 6.

2. Formulation according to claim 1, whereby the fat soluble vitamin is selected from the group of vitamin A and/or its derivatives and/or vitamin D and/or its dervatives and/or vitamin K and/or its derivatives.

3. Formulation according to claim 1, whereby the carotenoid is selected from *β*-carotene, lutein, lutein ester or lycopene and mixtures thereof.

4. Formulation according to claim 3, whereby the fat soluble vitamin is selected from the group of vitamin A and/or its derivatives.

5. Formulation according to claim 4, whereby the fat soluble vitamin is vitamin A palmitate.

6. Formulation according to claims 1 to 5, whereby the hydrocolloid is selected from polysaccharides.

7. Formulation according to claim 6, whereby the polysaccharide is selected from xanthan gum, acacia gum, pectin, guar gum, caroub gum, alginate, cellulose, cellulose derivatives, starch, starch derivatives and/or modified food starch.

8. Formulation according to claim 7, whereby the modified food starch is starch sodium octenyl succinate.

9. Formulation according to claims 1 to 8, whereby the starch hydrolysate has a DE-value of 10 to 30.

10. Formulation according to claim 9, whereby the starch hydrolysate has a DE-value of 15 to 25.

11. Formulation according to claim 9, whereby the starch hydrolysate has a DE-value of 17 to 20.

12. Use of a formulation according to claims 1 to 11 for staple food fortification.

13. Use according to claim 12 whereby the food fortification is a flour-, sugar-, rice- or condiment-fortification.

## Patentansprüche

1. Pulverhaltige, BHT-freie Formulierung, die 1 bis 25 Gewicht des Gesamtgewichts eines fettlöslichen Vitamins oder Carotenoids, 9,25 bis 25 Gewicht des Pulvers eines Hydrokolloids, 20 bis 89 Gewicht des Gesamtgewichts des Pulvers eines Stärkehydrolysats und 0,75 bis 12 Gewicht des Gesamtgewichts des Pulvers aus Tocopherol und Natriumascorbat ausmacht, optional 0 bis 20 Gewicht % des Gesamtgewichts des Pulvers des Trägeröls, optional 0 bis 50 Gewicht des Gesamtgewichts des Pulvers aus einer Mischung aus Pulver und Verkalkungsmittel und optional 0 bis 5 Gewicht des Gesamtgewichts des Restwasserpulvers, wobei das Gewichtsverhältnis von Natriumascorbat zu Tocopherol zwischen 1 und 6 liegt, Am besten zwischen 1,5 und 6.

2. Formulierung nach Anspruch 1, bei der das fettlösliche Vitamin aus der Gruppe von Vitamin A und/oder dessen Derivaten und/oder Vitamin D und/oder dessen Derivaten und/oder Vitamin K und/oder dessen Derivaten ausgewählt wird.

3. Formulierung gemäß Anspruch 1, bei der das Carotenoid aus β-Carotin, Lutein, Luteinester oder Lycopin und deren Mischungen ausgewählt wird.

4. Formulierung nach Anspruch 3, bei der das fettlösliche Vitamin aus der Gruppe von Vitamin A und/oder deren Derivaten ausgewählt wird.

5. Formulierung gemäß Behauptung 4, wobei das fettlösliche Vitamin Vitamin A-Palmitat ist.

6. Formulierung gemäß Ansprüche 1 bis 5, wobei das Hydrokolloid aus Polysacchariden ausgewählt wird.

7. Formulierung gemäß Anspruch 6, bei der das Polysaccharid aus Xanthangummi, Akaziengummi, Pektin, Guargummi, Johannesfettgummi, Alginat, Cellulose, Zellulosederivaten, Stärke, Stärkederivaten und/oder modifizierter Lebensmittelstärke ausgewählt wird.

8. Formulierung gemäß Anspruch 7, wobei die modifizierte Lebensmittelstärke Stärke Stärke ist Natrium-Octenylsuccinat.

9. Formulierung nach Angaben 1 bis 8, wobei das Stärkehydrolysat einen DE-Wert von 10 bis 30 hat.

10. Formulierung gemäß Anspruch 9, wobei das Stärkehydrolysat einen DE-Wert von 15 bis 25 hat.

11. Formulierung nach Anspruch 9, wobei das Stärkehydrolysat einen DE-Wert von 17 bis 20 hat.

12. Verwendung einer Formulierung gemäß Angaben 1 bis 11 zur Aufreichung von Grundnahrungsmitteln.

13. Verwendung gemäß Behauptung 12, wobei die Lebensmittelanreicherung eine Mehl-, Zucker-, Reis- oder Gewürzanreicherung ist.

## Revendications

1. Formulation poudre sans BHT comprenant 1 à 25 % du poids total de la poudre d'une vitamine liposoluble ou d'un caroténoïde, 9,25 à 25 % du poids total de la poudre d'un hydrocolloïde, 20 à 89 % du poids total de la poudre d'un hydrolysat d'amidon et 0,75 à 12 % du poids total de la poudre de tocophérol et d'ascorbate sodique, optionnellement 0 à 20 % en poids total de la poudre d'huile porteuse, optionnellement 0 à 50 % du poids total de la poudre d'un mélange d'agent poudre et anti-compressant, et optionnellement 0 à 5 % en poids total de la poudre d'eau résiduelle, le rapport de poids entre l'ascorbate de sodium et le tocophérol étant compris entre 1 et 6, De préférence entre 1,5 et 6.

2. Formulation selon la revendication 1, selon laquelle la vitamine liposoluble est sélectionnée parmi le groupe de la vitamine A et/ou de ses dérivés et/ou de la vitamine D et/ou de ses dérivés et/ou de la vitamine K et/ou de ses dérivés.

3. Formulation selon la revendication 1, selon laquelle le caroténoïde est sélectionné parmi β-carotène, lutéine, ester de lutéine ou lycopène et leurs mélanges.

4. Formulation selon la revendication 3, selon laquelle la vitamine liposoluble est sélectionnée parmi le groupe de vitamines A et/ou ses dérivés.

5. Formulation selon la revendication 4, selon laquelle la vitamine liposoluble est la vitamine A palmité.

6. Formulation selon les revendications 1 à 5, où l'hydrocolloïde est sélectionné parmi les polysaccharides.

7. Formulation selon la revendication 6, selon laquelle le polysaccharide est sélectionné à partir de gomme xanthane, gomme d'acacia, pectine, gomme guar, gomme caroubie, alginate, cellulose, dérivés de cellulose, amidon, dérivés d'amidon et/ou amidon alimentaire modifié.

8. Formulation selon la revendication 7, selon laquelle l'amidon alimentaire modifié est de l'amidon octényle sodique.

9. Formulation selon les revendications 1 à 8, selon laquelle l'hydrolysat d'amidon a une valeur DE de 10 à 30.

10. Formulation selon l'affirmation 9, selon laquelle l'hydrolysat d'amidon a une valeur DE de 15 à 25.

11. Formulation selon la revendication 9, selon laquelle l'hydrolysat d'amidon a une valeur DE de 17 à 20.

12. Utilisation d'une formulation selon les revendications 1 à 11 pour la fortification des aliments de base.

13. Utilisation selon la revendication 12 selon laquelle la fortification alimentaire est une fortification de farine, sucre, riz ou condiment.
